**Europäisches Patentamt**

**European Patent Office**

**Office européen des brevets**

(19)

(11) Veröffentlichungsnummer: **0 010 710**
**B1**

(12) # EUROPÄISCHE PATENTSCHRIFT

(45) Veröffentlichungstag der Patentschrift:
**23.06.82**

(21) Anmeldenummer: **79104076.9**

(22) Anmeldetag: **22.10.79**

(51) Int. Cl.³: **C 07 C 69/96**, C 07 C 68/02

(54) Verfahren zur Herstellung von Chlorameisensäuremethylester.

(30) Priorität: **02.11.78 DE 2847484**

(43) Veröffentlichungstag der Anmeldung:
**14.05.80 Patentblatt 80/10**

(45) Bekanntmachung des Hinweises auf die Patenterteilung:
**23.06.82 Patentblatt 82/25**

(84) Benannte Vertragsstaaten:
**DE FR GB**

(56) Entgegenhaltungen:
**DE-A1-2 545 656**
**DE-A1-2 704 262**
**DE-B2-2 159 967**

(73) Patentinhaber: **BAYER AG, Zentralbereich Patente, Marken und Lizenzen, D-5090 Leverkusen 1, Bayerwerk (DE)**

(72) Erfinder: **Schulte-Huermann, Werner, Dr., Helmut-Macke-Strasse 10, D-4150 Krefeld (DE)**
Erfinder: **Schellmann, Erhard, Dr., An der Ruthen 1, D-5000 Köln 80 (DE)**
Erfinder: **Lahrs, Jürgen, Dr., Volberger Weg 11, D-5000 Köln 91 (DE)**

## Verfahren zur Herstellung von Chlorameisensäuremethylester

Die Erfindung betrifft ein Verfahren zur Herstellung von Chlorameisensäuremethylester durch Umsetzung von Phosgen mit Methanol. Die Herstellung von Chlorameisensäuremethylester durch Einleiten von Phosgen in Methanol im Temperaturbereich von 0 bis 10°C ist bekannt (Houben-Weyl, Band 8, 101 ff, 1952).

In der DE-OS 2 251 206 wird die kontinuierliche Herstellung von Chlorameisensäuremethylester beschrieben, bei der man den Alkohol, vorzugsweise mittels einer Strahldüse, und Phosgen einem Reaktionsgefäß, daß im zeitlichen Gleichgewicht wenigstens 20 Gew.-% Phosgen im Reaktionsgemisch enthält, derart zuführt, daß die Zeit für die homogene Einmischung des Alkohols in das Reaktionsgemisch weniger als 5 Sekunden beträgt, eine den zugeführten Mengen an Alkohol und Phosgen entsprechende Menge des Reaktionsgemischs abzieht, dieses von Phosgen und Chlorwasserstoff befreit und das Phosgen dem Reaktionsgefäß wieder zuführt.

Aus der DE-OS 2 453 284 ist bekannt, daß bei der Herstellung von Chlorameisensäuremethylester die Zeit für die homogene Einmischung des Alkohols weniger als 1 Sekunde beträgt, wobei im zeitlichen Gleichgewicht ein Phosgenüberschuß von nur mehr wenigstens 2 Gew.-% im Reaktionsgemisch, eine Temperatur von bis zu 70°C und eine Verweilzeit bis zu 300 Minuten eingehalten werden kann.

Nach dem in der DE-OS 2 704 262 beschriebenen Verfahren zur Herstellung von Chlorameisensäuremethylester wird das flüssige Methanol rasch in den im Kreislauf befindlichen Chlorameisensäuremethylester dispergiert (siehe Seite 7, 8). Das Phosgen wird von dem zirkulierenden Reaktionsgemisch in einem Absorptionssturm, der mit Raschigringen aus Glas oder einem anderen nicht katalytisch wirkenden Füllkörpermaterial gefüllt ist, im Gegenstrom absorbiert (Seite 8, Zeilen 13 bis 16). Es wird ferner beschrieben (Seite 2, Zeilen 2 bis 3), daß man das flüssige Phosgen im Chlorameisensäuremethylester dispergiert.

Es wurde ein Verfahren zur kontinuierlichen Herstellung von Chlorameisensäuremethylester durch Umsetzung von Phosgen mit Methanol bei einer Temperatur bis zu 50°C in einem Reaktor, der Chlorameisensäuremethylester als Lösungsmittel enthält, und in dem Phosgen dispergiert wird, gefunden, das dadurch gekennzeichnet ist, daß man gasförmiges Phosgen mit gegebenenfalls bereits gebildetem Chlorameisensäuremethylester in dem Reaktionsraum mittels einer Strahldüse dispergiert und gleichzeitig Methanol in die homogene Gas-Flüssig-Dispersion in der Weise zugibt, daß im Reaktionsraum unabhängig von der Ausgangstemperatur die Reaktionstemperatur um höchstens 10°C ansteigt.

Im allgemeinen wird Chlorameisensäuremethylester aus Methanol und einem Überschuß Phosgen in einem Kreislaufprozeß hergestellt. Als Lösungsmittel wird Chlorameisensäuremethylester vorgelegt. Wesentliche apparative Bestandteile dieses Kreislaufes sind der Reaktor, der Wärmetauscher und die Zugabe- und Entnahmevorrichtungen der Reaktanden und der Reaktionsprodukte Chlorameisensäuremethylester und Chlorwasserstoff.

Nach dem erfindungsgemäßen Verfahren wird das gasförmige Phosgen in den Reaktionsraum mittels einer Strahldüse fein ohne Zeitbegrenzung dispergiert. Gleichzeitig wird das Methanol feinverteilt in die Gas-Flüssig-Dispersion gegeben. Es ist wesentlich für das erfindungsgemäße Verfahren, daß im Reaktionsraum eine homogene Gas-Flüssig-Dispersion von Phosgen und dem Reaktionsgemisch, im wesentlichen Chlorameisensäuremethylester, sowie Methanol entsteht.

Das erfindungsgemäße Verfahren kann in dem für die Herstellung von Chlorameisensäuremethylester aus Methanol und Phosgen üblichen Temperaturbereich, vorzugsweise von 0 bis 30°C durchgeführt werden. Es ist jedoch wesentlich, daß hierbei im Reaktionsraum unabhängig von der Ausgangstemperatur die Reaktionstemperatur um höchstens 10°C, vorzugsweise um höchstens 5°C, ansteigt. Der Temperaturanstieg im Reaktionsraum kann nach dem erfindungsgemäßen Verfahren durch die Menge des im Kreislauf geförderten Chlorameisensäuremethylesters gesteuert werden. Vorzugsweise erreicht man eine Temperatursteuerung innerhalb der erfindungsgemäßen Grenzen, wenn 1 Mol Methanol mit 1 bis 1,3 Mol, vorzugsweise 1 bis 1,1 Mol Phosgen, in 5 bis 20 Mol, vorzugsweise 10 bis 15 Mol Chlorameisensäuremethylester als Lösungsmittel umgesetzt wird.

Vorzugsweise befindet sich nach dem erfindungsgemäßen Verfahren der Reaktionsraum in dem unteren Teil einer Blasensäule, die als Nachreaktionsraum nachgeschaltet ist. Blasensäulen, wie sie für das erfindungsgemäße Verfahren verwendet werden können, sind an sich bekannt (siehe Ullmann, (1972), Bd. 1, S. 228 ff).

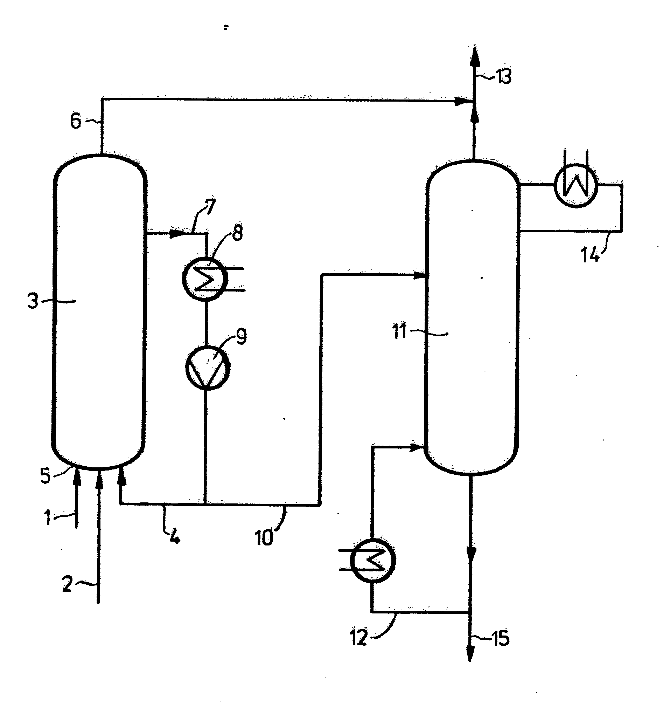

Das erfindungsgemäße Verfahren kann beispielsweise, wie anhand der Zeichnung 1 erläutert, durchgeführt werden: Gasförmiges Phosgen (1) wird mit einer großen Menge Chlorameisensäuremethylester (4) am Boden der Blasensäule (3) mit einer Strahldüse (5) fein dispergiert. Gleichzeitig wird in diese Gas-Flüssigkeits-Dispersion gekühltes Methanol (2) eingegeben. Der entstehende Chlorwasserstoff (6) strömt am Kopf der Blasensäule ab. Das Reaktionsgemisch (7) wird über einen Überlauf dem Kühler (8) zugeleitet, in dem die Reaktionswärme abgeführt wird. Die Pumpe (9) wälzt den Chlorameisensäuremethylester um, der noch mit Chlorwasserstoff und Phosgen beladen ist. Ein Teilstrom des Esters (10) gelangt in die Entgasungskolonne (11), in deren Sumpf bei etwa 71°C der Chlorameisensäuremethylester verdampft wird (12). Am Kopf der Kolonne (13) strömen Chlorwasserstoff und Phosgen als Abgas über einen Kühler (14) ab, in dem der Chlorameisensäuremethylester kondensiert wird. Der Chlorameisensäuremethylester wird im Teilstrom am Boden (15) der Kolonne (11) entnommen.

Nach dem erfindungsgemäßen Verfahren erhält man Chlorameisensäuremethylester mit hoher Reinheit und mit großen Ausbeuten. Es ist überraschend, daß sich Chlorameisensäuremethylester mit Hilfe des erfindungsgemäßen Verfahrens mit geringem Dimethylcarbonat- und Methylchloridanteil herstellen läßt. Nach dem erfindungsgemäßen Verfahren ist es daher nicht erforderlich, angefallenes Dimethylcarbonat mit Wasser zu extrahieren und den Chlorameisensäuremethylester zu trocknen. Es entfällt auch die Aufarbeitung des als Abwasser anfallenden Dimethylcarbonat enthaltenden Wassers.

Die Erzeugung einer feinen Gas-Flüssig-Dispersion aus Phosgen und Chlorameisensäuremethylester sowie das gleichzeitige Eingeben von Methanol in diese Dispersion bringt außerdem einen überraschend guten Reaktionsablauf. Es zeigt sich in nicht zu erwartender Weise, daß die sonst gemachten Anstrengungen für kurze Mischzeiten und die Benutzung von flüssigem Phosgen nicht entscheidend sind. Vielmehr ist die Reaktionszeit unabhängig von der Mischzeit.

Es ist weiter überraschend, daß die Nachreaktion von vorhandenem Ester mit Methanol so stark zurückgedrängt wird, obwohl das gasförmig eindispergierte Phosgen über seine Löslichkeit im Chlorameisensäuremethylester erst zur Reaktion mit Methanol zur Verfügung steht.

Der bei der Reaktion entstehende Chlorwasserstoff reagiert überraschenderweise nicht mit dem Chlorameisensäuremethylester zu Methylchlorid.

Der besondere Vorteil des erfindungsgemäßen Verfahrens besteht darin, daß bei nur geringen Phosgenüberschüssen ohne technischen Aufwand für besonders kurze Mischzeiten in einem bekannten Reaktor (Blasensäule) die Herstellung von Chlorameisensäuremethylester mit großer Reinheit möglich ist.

Chlorameisensäuremethylester ist ein Zwischenprodukt für Pflanzenschutzmittel und Farbstoffe (Ullmann, Bd. 9, S. 382 ff. (1972)).

## Beispiel

Entsprechend der Zeichnung 1 werden 325 kg/h Phosgen (1) mit 12 000 kg/h Chlorameisensäuremethylester (4) am Boden der Blasensäule (3) mit einer Strahldüse (5) fein dispergiert. Gleichzeitig wird in diese Gas-Flüssigkeits-Dispersion 103 kg/h gekühltes Methanol (2) eingegeben. Der entstehende Chlorwasserstoff (6) strömt am Kopf der Blasensäule ab. Das Reaktionsgemisch (7) wird über einen Überlauf dem Kühler (8) zugeleitet, in dem die Reaktionswärme abgeführt wird. Die Pumpe (9) wälzt etwa 10 m³/h Chlorameisensäuremethylester um, der teilweise noch mit Chlorwasserstoff und Phosgen beladen ist. Ein Teilstrom des Esters (10) gelangt in die Entgasungskolonne (11), in deren Sumpf bei etwa 71°C der Chlorameisensäuremethylester verdampft wird (12). Am Kopf der Kolonne (13) strömen der Chlorwasserstoff und das Phosgen als Abgas über einen Kühler (14) ab, in dem der Chlorameisensäuremethylester kondensiert wird. 300 kg/h Chlorameisensäuremethylester werden im Teilstrom am Boden (15) der Kolonne (11) entnommen.

Der Dimethylcarbonatgehalt des Chlorameisensäuremethylesters ist kleiner als 1 Gew.-%.

## Patentansprüche

1. Verfahren zur kontinuierlichen Herstellung von Chlorameisensäuremethylester durch Umsetzung von Phosgen mit Methanol bei einer Temperatur bis zu 50°C in einem Reaktor der Chlorameisensäuremethylester als Lösungsmittel enthält und in dem Phosgen dispergiert wird, dadurch gekennzeichnet, daß man gasförmiges Phosgen mit gegebenenfalls bereits gebildetem Chlorameisensäuremethylester in dem Reaktionsraum mittels einer Strahldüse dispergiert und gleichzeitig Methanol in die homogene Gas-Flüssig-Dispersion in der Weise zugibt, daß im Reaktionsraum unabhängig von der Ausgangstemperatur die Reaktionstemperatur um höchstens 10°C ansteigt.

2. Verfahren nach Anspruch 1, dadurch gekennzeichnet, daß die Erhöhung der Temperatur im Reaktionsraum durch die umlaufende Menge des Chlorameisensäuremethylesters gesteuert wird.

3. Verfahren nach den Ansprüchen 1 und 2, dadurch gekennzeichnet, daß als Reaktionsraum eine Blasensäule verwendet wird.

4. Verfahren nach den Ansprüchen 1, 2 und 3, dadurch gekennzeichnet, daß die zur Dispergierung des Phosgens notwendige umgewälzte Estermenge eine Erwärmung des Reaktionsgemischs um maximal 10°C zuläßt.

## Claims

1. Process for the continuous preparation of chloroformic acid methyl ester by reacting phosgene with methanol at a temperature of up to 50°C in a reactor which contains chloroformic acid methyl ester as the solvent and in which phosgene is dispersed, characterised in that gaseous phosgene, together with any chloroformic acid methyl ester which has already formed, is dispersed in the reaction chamber by means of a spray nozzle, and at the same time methanol is introduced into the homogeneous gas-in-liquid dispersion in such a way that the reaction temperature in the reaction chamber rises by at most 10°C independently of the starting temperature.

2. Process according to Claim 1, characterised in that the rise in temperature in the reaction chamber is controlled by the amount of chloroformic acid methyl ester circulating.

3. Process according to Claims 1 and 2, characterised in that a bubble column is used as the reaction chamber.

4. Process according to Claims 1, 2 and 3, characterised in that the amount of circulating ester required for dispersing the phosgene permits a heating-up of the reaction mixture by at most 10°C.

## Revendications

1. Procédé de production continue d'ester méthylique d'acide chloroformique par réaction de phosgène avec le méthanol à une température allant jusqu'à 50°C dans un réacteur qui contient de l'ester méthylique d'acide chloroformique comme solvant et dans lequel du phosgène est dispersé, caractérisé en ce qu'on disperse du phosgène gazeux avec de l'ester méthylique d'acide chloroformique éventuellement déjà formé dans la chambre de réaction au moyen d'une buse d'injection et on ajoute en même temps du méthanol dans la dispersion gaz-liquide homogène de telle sorte que la température de réaction s'élève au maximum de 10°C dans la chambre de réaction, indépendamment de la température initiale.

2. Procédé suivant la revendication 1, caractérisé en ce que l'élévation de température dans la chambre de réaction est réglée par la quantité en circulation d'ester méthylique d'acide chloroformique.

3. Procédé suivant les revendications 1 et 2, caractérisé en ce qu'on utilise une colonne à bulles comme chambre de réaction.

4. Procédé suivant les revendications 1, 2 et 3, caractérisé en ce que la quantité d'ester en circulation nécessaire à la dispersion du phosgène permet un échauffement du mélange réactionnel de 10°C au maximum.